# EUROPÄISCHE PATENTANMELDUNG

(11) **EP 1 281 732 A1**
(43) Veröffentlichungstag der Anmeldung: **05.02.2003**
(21) Anmeldenummer: 02014910.0
(22) Anmeldetag: 05.07.2002
(51) Int. Cl.: C09C 1/00

(54) **Mehrschichtige Interferenzpigmente**

(30) Priorität: 02.08.2001 DE 10137831
(71) Anmelder: MERCK PATENT GmbH, 64293 Darmstadt (DE)
(72) Erfinder: Pfaff, Gerhard, Dr., 64839 Münster (DE); Kniess, Helge, 64331 Weiterstadt (DE); Reynders, Peter, Dr., 64347 Griesheim (DE)

(57) **Zusammenfassung**

Die Erfindung betrifft mehrschichtige Interferenzpigmente, welche ein plättchenförmiges Substrat aus Siliziumdioxid umfassen, welches abwechselnd mit Schichten aus lichtdurchlässigen Materialien mit Brechzahlen von n > 1,8 und Schichten aus lichtdurchlässigen Materialien mit Brechzahlen von n ≤ 1,8 beschichtet ist, wobei die optische Dicke des plättchenförmigen Substrates und die optische Dicke der einzelnen Schichten aus lichtdurchlässigen Materialien mit Brechzahlen von n ≤ 1,8 gleich ist.

## Beschreibung

Die Erfindung betrifft mehrschichtige Interferenzpigmente, welche ein plättchenförmiges Substrat aus Siliziumdioxid umfassen, welches abwechselnd mit Schichten aus lichtdurchlässigen Materialien mit Brechzahlen von n > 1,8 und Schichten aus lichtdurchlässigen Materialien mit Brechzahlen von n ≤ 1,8 beschichtet ist, wobei die optische Dicke des plättchenförmigen Substrates und die optische Dicke der einzelnen Schichten aus lichtdurchlässigen Materialien mit Brechzahlen von n ≤ 1,8 gleich ist, sowie ein Verfahren zur Herstellung dieser Pigmente und deren Verwendung.

Der Einsatz von Glanz- oder Effektpigmenten, die Interferenzerscheinungen aufweisen, ist weit verbreitet. Bei Automobillacken, dekorativen Beschichtungen aller Art sowie bei der Einfärbung von Kunststoffen, Anstrich- und Druckfarben, insbesondere Farben für den Sicherheitsdruck, sowie bei Anwendungen in der dekorativen Kosmetik sind derartige Pigmente unverzichtbar geworden.
In der sie umgebenden Matrix richten sich diese Pigmente in idealer Weise parallel zur Oberfläche der Beschichtung aus und entfalten durch ein kompliziertes Zusammenspiel von Interferenz, Reflexion und Absorption des auffallenden Lichtes ihre optische Wirkung. Dabei stehen für die verschiedenen Anwendungsfälle eine brillante Farbgebung, Wechsel zwischen verschiedenen Farben in Abhängigkeit vom Betrachtungswinkel, so genannte Farbflops, oder wechselnde Helligkeitseindrücke im Mittelpunkt des Interesses.

Die Herstellung solcher Pigmente erfolgt in der Regel durch Beschichtung von plättchenförmigen metallischen oder nichtmetallischen Substraten mit Metalloxid- oder Metallschichten. In den letzten Jahren sind verstärkt Pigmente entwickelt worden, welche auf dem plättchenförmigen Träger einen Mehrschichtaufbau aufweisen. Hierdurch lassen sich vor allem Farbflops, bei denen das menschliche Auge abhängig vom Betrachtungswinkel verschiedene Farbtöne wahrnimmt, gezielt einstellen. Die meisten dieser Pigmente basieren auf plättchenförmigen Substraten aus Metallen oder künstlichen oder natürlichen Schichtsilikaten, wie Glimmer, Talk oder Glas. Dabei weisen insbesondere die Schichtsilikate den Nachteil auf, dass die Dicke des Substrates in einem weiten Bereich variiert und nicht gezielt einstellbar ist, was dazu führt, dass auch bei transparenten Substraten Lichttransmission und -reflexion am Substrat weitgehend unkontrollierbar ablaufen und daher nicht gezielt nutzbar sind.

Es sind jedoch auch Interferenzpigmente mit Substraten aus Siliziumdioxid bekannt. So werden zum Beispiel in DE 41 37 764 Pigmente auf der Basis von Siliziumdioxidpartikeln beschrieben, welche mit Eisenoxid beschichtet sind, wobei der auf die Siliziumdioxidpartikel bezogene Eisenoxidanteil mehr als 350 Gew. -% beträgt. Solche Pigmente weisen ein gutes Deckvermögen, kräftige Farbtöne sowie einen Farbflop auf, obwohl sie nicht auf plättchenförmigen Substraten basieren. Dabei wird der Farbflop mit steigendem Eisenoxidanteil ausgeprägter. Für Pigmentanwendungen, bei denen ein Farbflop erwünscht ist, müssen deshalb sehr dicke Eisenoxidschichten aufgebracht werden. So hergestellte Pigmente lassen durch die Eisenoxidbeschichtung nur einen begrenzten Umfang an Farbtönen zu und sind in Präparationen, bei denen Transparenz und dünne Schichtdicken erforderlich sind, nur bedingt anwendbar. Ein großer Teil des auffallenden Lichtes kann außerdem nicht optimal reflektiert werden, da sich die Pigmente in Beschichtungen nicht parallel zur Oberfläche orientieren können.

Aus EP 0 608 388 ist ein plättchenförmiges Pigment bekannt, welches u. a. aus einer Siliziumdioxidmatrix bestehen kann, welche mit einer oder mehreren Schichten aus dünnen, transparenten oder semitransparenten reflektierenden Schichten aus Metalloxiden oder Metallen beschichtet ist. Dabei wird die Matrix bevorzugt durch Zugabe von verschiedenen Farbmitteln eingefärbt. Es werden Pigmente mit großer Farbreinheit und hoher Farbstärke erhalten, deren Deckvermögen durch den Grad der Einfärbung der Matrix bestimmt wird. Die Dicke der Matrix kann dabei in einem breiten Bereich eingestellt werden. Es sind dort keine Pigmente beschrieben worden, die mehr als eine optisch aktive Schicht auf dem Substrat aufweisen. Diese Pigmente besitzen daher die Wirkung eines Pigmentes mit insgesamt drei optisch aktiven Schichten.

In DE 196 14 637 werden goniochromatische Glanzpigmente auf der Basis von Siliziumdioxidplättchen offenbart, auf welchen mittels eines CVD-Verfahrens eine schwarze, mindestens teilweise transparente Schicht (A) und optional eine äußere Schicht (B), welche aus einem farblosen oder selektiv absorbierenden Metalloxid besteht und/oder phosphat-, chromatund /oder vanadathaltig ist, aufgebracht ist.
Dabei besteht die 1 bis 50 nm dicke Schicht (A) vorzugsweise aus Metallen und/oder nichtselektiv absorbierenden Metallverbindungen. Die Schicht (B) kann aus hoch- oder niedrigbrechenden Metalloxiden bestehen.
Durch die schwarze Schicht (A) zeigen diese Pigmente intensive Interferenzfarben und auffällige Farbtonänderungen in Abhängigkeit vom Betrachtungswinkel.
Metall- und Metalloxidschichten werden hier jedoch über die Gasphasenzersetzung von insbesondere metallorganischen Verbindungen aufgebracht, wodurch das Herstellungsverfahren für die Massenproduktion von Pigmenten zu aufwändig und kostspielig und mit hohen Sicherheitsvorkehrungen verbunden ist.

Die DE 196 18 569 betrifft mehrschichtige Interferenzpigmente aus einem transparenten Trägermaterial, welches mit alternierenden Schichten eines Metalloxides mit niedriger Brechzahl und eines Metalloxides mit hoher Brechzahl beschichtet ist.
Dabei kann das transparente Trägermaterial aus SiO₂-Plättchen bestehen. In einem Beispiel werden auf ein SiO₂-Plättchen abwechselnd TiO₂, SiO₂ und TiO₂ aufgebracht, wobei die einzelnen Schichtdicken sehr unterschiedlich sind. Bei diesen Pigmenten werden der Interferenzfarbton bei senkrechter Betrachtung sowie Anzahl und Farbtöne der abhängig vom Betrachtungswinkel durchschrittenen Farben (Farbflop) sowie der Buntton (Farbstärke) des Pigmentes erst dann sichtbar, wenn der endgültige Pigmentaufbau erreicht ist. In diesem Falle sind Farbkorrekturen aber nur noch in der äußeren Schicht vornehmbar. Das ist in der Regel nur durch den Auftrag von höheren Schichtdicken erreichbar, was wirtschaftlich ungünstig ist und in Extremfällen dazu führen kann, dass das Produkt allein auf Grund der höheren Schichtdicken für bestimmte Anwendungszwecke nicht mehr einsetzbar ist.
Auch ist der Buntton solcher Pigmente bei den unterschiedlichen Farben eines Farbflops unterschiedlich stark ausgeprägt, das heißt die verschiedenen Farben werden mit unterschiedlicher Intensität wahrgenommen und besitzen keine ausgeprägte Brillanz.

Es bestand daher ein Bedarf an mehrschichtigen Interferenzpigmenten, die intensive Interferenzfarben und eine starke Winkelabhängigkeit der Interferenzfarbe aufweisen, sowie an einem einfachen und kostengünstigen Herstellungsverfahren, mit welchem bereits in der ersten Verfahrensstufe die Farbeigenschaften des Endproduktes maßgeblich bestimmt werden können.

Es ist daher die Aufgabe der Erfindung, weitere mehrschichtige Interferenzpigmente auf der Basis von Siliziumdioxidplättchen zur Verfügung zu stellen, die eine starke Winkelabhängigkeit der Interferenzfarben besitzen, wobei alle bei der Änderung des Betrachtungswinkels durchlaufenen Farbtöne einen intensiven Buntton sowie eine hervorragende Brillanz aufweisen.
Weiterhin war es die Aufgabe der Erfindung, ein Verfahren zur Herstellung von mehrschichtigen Interferenzpigmenten bereitzustellen, bei welchem bereits mit der ersten Beschichtung des Substrates die bei verschiedenen Betrachtungswinkeln zu durchlaufenden verschiedenen Interferenzfarbtöne verbindlich eingestellt werden können.
Eine zusätzliche Aufgabe der Erfindung bestand darin, Verwendungen für die erfindungsgemäßen Pigmente aufzuzeigen.

Die Aufgabe der Erfindung wird durch ein mehrschichtiges Interferenzpigment gelöst, das ein Substrat aus plättchenförmigem Siliziumdioxid umfasst, welches abwechselnd mit zwei oder mehreren Schichten aus lichtdurchlässigen Materialien mit Brechzahlen von n > 1,8 und einer oder mehreren Schichten aus lichtdurchlässigen Materialien mit Brechzahlen von n ≤ 1,8 beschichtet ist, wobei die optische Dicke des plättchenförmigen Substrates und die optische Dicke der einzelnen Schichten aus lichtdurchlässigen Materialien mit Brechzahlen von n ≤ 1,8 gleich ist.

Die Aufgabe der Erfindung wird ebenfalls gelöst durch ein Verfahren zur Herstellung eines mehrschichtigen Interferenzpigmentes, wobei ein plättchenförmiges Substrat aus Siliziumdioxid in Wasser suspendiert und abwechselnd mehrfach mit einem Metalloxidhydrat oder BiOCI mit einer Brechzahl von n > 1,8 und einem Metalloxidhydrat oder Metallfluorid mit einer Brechzahl von n ≤ 1,8 durch Zugabe und Fällung der entsprechenden, anorganischen Metallverbindungen beschichtet wird, wobei durch gleichzeitige Zugabe von Säure oder Base der für die Fällung des jeweiligen Metalloxidhydrates, BiOCI oder Metallfluorides notwendige pH-Wert eingestellt und konstant gehalten wird und anschließend das beschichtete Substrat aus der wässrigen Suspension abgetrennt, getrocknet und gegebenenfalls kalziniert wird, und wobei bei den einzelnen auf dem Substrat aufzubringenden Schichten aus einem Metalloxidhydrat oder Metallfluorid mit einer Brechzahl von n ≤ 1,8 eine optische Schichtdicke so eingestellt wird, dass sie nach dem Trocknen und gegebenenfalls Kalzinieren gleich der optischen Schichtdicke des plättchenförmigen Substrates ist.

Weiterhin wird die Aufgabe der Erfindung gelöst durch die Verwendung der oben beschriebenen erfindungsgemäßen mehrschichtigen Interferenzpigmente zur Pigmentierung von Farben, Lacken, Druckfarben, Kunststoffen, kosmetischen Formulierungen, Gläsern, Papier, Folien, Verpackungsmaterialien, keramischen Materialien, zur Lasermarkierung, in Sicherheitsanwendungen sowie in Trockenpräparaten und Pigmentpräparationen.

Das Substrat des erfindungsgemäßen Interferenzpigmentes besteht aus plättchenförmigen Siliziumdioxidpartikeln, welche über eine einheitliche Schichtdicke verfügen und vorzugsweise gemäß der internationalen Anmeldung WO 93/08237 auf einem endlosen Band durch Verfestigung und Hydrolyse einer Wasserglaslösung hergestellt werden. Unter einheitlicher Schichtdicke wird dabei eine Schichtdickentoleranz von 3 bis 10%, bevorzugt von 3 bis 5 % der Gesamttrockenschichtdicke der Partikel verstanden. Die plättchenförmigen Siliziumdioxidpartikel liegen im allgemeinen in amorpher Form vor.
Der Durchmesser der SiO₂-Plättchen liegt üblicherweise zwischen 1 und 250 µm, vorzugsweise zwischen 2 und 100 µm. Ihre Dicke beträgt zwischen 100 und 600 nm, bevorzugt 200 bis 500 nm und besonders bevorzugt 200 bis 375 nm.
Das mittlere Aspektverhältnis der plättchenförmigen SiO₂-Partikel, das heißt das Verhältnis vom mittleren Längenmesswert, der hier dem mittleren Durchmesser entspricht, zum mittleren Dickenmesswert, beträgt üblicherweise 5 bis 200, bevorzugt 20 bis 150 und besonders bevorzugt 30 bis 120.

Als lichtdurchlässige Materialien mit Brechzahlen von n ≤ 1,8 kommen Metalloxide, Metallfluoride oder Metalloxidhydrate, die vorzugsweise farblos sind, wie z.B. SiO₂, SiO(OH)₂, Al₂O₃, AIO(OH), B₂O₃, MgF₂ oder deren Gemische, in Betracht.
Besonders bevorzugt ist SiO₂.

Die Dicke der einzelnen Schichten aus diesen Materialien beträgt zwischen 100 und 600 nm, bevorzugt 200 bis 500 nm und besonders bevorzugt 200 bis 375 nm für SiO₂- Schichten sowie 50 bis 600 nm, bevorzugt 80 bis 400 nm und besonders bevorzugt 100 bis 350 nm für die anderen Materialien. Dabei wird die Dicke der Schichten derart eingestellt, dass die optische Schichtdicke des Substrates gleich der optischen Schichtdicke jeder einzelnen Schicht aus lichtdurchlässigen Materialien mit Brechzahlen von n ≤ 1,8 ist. Unter gleicher optischer Schichtdicke wird dabei verstanden, dass die Abweichung zwischen der optischen Schichtdicke des Substrates und der optischen Schichtdicke jeder einzelnen Schicht aus lichtdurchlässigen Materialien mit Brechzahlen n ≤ 1,8 maximal 5 %, bevorzugt jedoch maximal 3 %, bezogen auf die optische Schichtdicke des Substrates, beträgt. Die optische Schichtdicke der einzelnen Schichten bestimmt sich dabei aus dem Produkt aus der Brechzahl n des Schichtmaterials und der tatsächlichen (physikalisch messbaren) Schichtdicke d. Ist das Material mit einer Brechzahl von n ≤ 1,8 Siliziumdioxid, ist die tatsächliche Dicke des Substrates gleich der tatsächlichen Dicke der Einzelschichten aus Siliziumdioxid. Ist das Material mit einer Brechzahl von n ≤ 1,8 jedoch SiO(OH)₂, Al₂O₃, AlO(OH), B₂O₃, MgF₂ oder ein Gemisch aus zwei oder mehreren von diesen bzw. ein Gemisch mit SiO₂ oder einem anderen lichtdurchlässigen Material mit einer Brechzahl von n ≤ 1,8, so wird die tatsächliche Schichtdicke dieser Schicht so eingestellt, dass ihre optische Schichtdicke der optischen Schichtdicke des Substrates entspricht.
Aus diesem Grunde unterliegen die tatsächlichen Schichtdicken der einzelnen Schichten aus lichtdurchlässigen Materialien mit Brechzahlen von n ≤ 1,8 gewissen Schwankungen, was auch für das Verhältnis der tatsächlichen Schichtdicken dieser einzelnen Schichten zur tatsächlichen Schichtdicke des Substrates zutrifft. So können die tatsächlichen Schichtdicken der einzelnen Schichten aus lichtdurchlässigen Materialien mit Brechzahlen von n ≤ 1,8 Schwankungen unterliegen, die kleiner oder gleich 20 %, bezogen auf die tatsächliche Schichtdicke des Substrates betragen, bevorzugt jedoch etwa 10 bis 20 %.
Das Verhältnis der Schichtdicke jeder Einzelschicht aus Materialien mit Brechzahlen von n ≤ 1,8 zur Schichtdicke des Substrates ist daher größer oder gleich 0,8 und kleiner oder gleich 1,2. Bevorzugt bestehen alle einzelnen Schichten aus lichtdurchlässigen Materialien mit Brechzahlen von n ≤ 1,8 aus demselben Material. Die Zusammensetzung der Schichten kann jedoch auch von Schicht zu Schicht verschieden sein oder es können sich Schichten aus zwei oder mehreren verschiedenen Materialien in gleich bleibender Reihenfolge abwechseln. In einer besonders bevorzugten Ausführungsform bestehen sowohl das Substrat als auch die einzelnen Schichten aus lichtdurchlässigen Materialien mit Brechzahlen von n ≤ 1,8 aus Siliziumdioxid.
Auf dem Substrat befinden sich eine oder mehrere solcher Schichten, insbesondere ein bis drei Schichten und besonders bevorzugt eine Schicht.

Als lichtdurchlässige Materialien mit Brechzahlen von n > 1,8 sind für Pigmentanwendungen bekannte Metalloxide, insbesondere farblose Metalloxide wie TiO₂, ZrO₂, ZnO oder SnO₂, deren Gemische oder auch BiOCI einsetzbar.
Besonders bevorzugt ist TiO₂.
Die Dicke dieser Schichten beträgt dabei jeweils etwa 20 bis 300 nm und bevorzugt 30 bis 200 nm.
Dabei ist die Dicke der einzelnen Schichten aus lichtdurchlässigen Materialien mit Brechzahlen von n > 1,8 stets kleiner als die Dicke der einzelnen Schichten aus lichtdurchlässigen Materialien mit Brechzahlen von n ≤ 1,8 oder die Dicke des Substrates.
Das erfindungsgemäße Mehrschichtpigment enthält zwei oder mehrere Schichten aus Materialien mit Brechzahlen n > 1,8, bevorzugt jedoch zwei Schichten. Diese Schichten können aus gleichen oder verschiedenen Materialien bestehen und gleiche oder verschiedene Schichtdicken aufweisen.
Bevorzugt bestehen diese Schichten aus den gleichen Materialien und weisen gleiche Schichtdicken auf. Das ist insbesondere dann der Fall, wenn die Schichtdicken des Substrates, der ersten Schicht aus einem lichtdurchlässigen Material mit einer Brechzahl von n > 1,8 sowie die Schichtdicke der ersten Schicht aus einem lichtdurchlässigen Material mit einer Brechzahl von ≤ 1,8 so aufeinander abgestimmt sind, dass von jeder Schicht das auftreffende Licht bestimmter Wellenlängen in gleichem Maße verstärkt wird, d.h. die optischen Schichtdicken der Beziehung (2n+1) λ/4 genügen, wobei n = 0,1,2,3... und λ die mittlere Wellenlänge des einstrahlenden Lichtes bedeutet. In diesem Falle spricht man von einer konstruktiven Interferenz. Es ist ebenfalls zu beachten, dass eine destruktive Interferenz, d.h. eine maximale Schwächung der Interferenz, welche bei optischen Schichtdicken auftritt, die der Beziehung n λ/2 genügen, vermieden wird.

Es können jedoch auch Anwendungsfälle auftreten, bei denen von diesem bekannten Prinzip optimaler Farbeinstellung abgewichen werden muss. So wird die Farb- und Glanzwirkung eines Interferenzpigmentes in hohem Grade von dem Medium bestimmt, in welchem das Pigment eingesetzt wird. Dasselbe Pigment zeigt zum Beispiel als Pigmentpulver an Luft andere Farbeigenschaften als in einer Bindemittelmatrix einer Beschichtungslösung. Aus diesem Grunde werden Interferenzpigmente auf ihr späteres Einsatzgebiet individuell zugeschnitten.
Soll nun das erfindungsgemäße mehrschichtige Interferenzpigment in einem Medium eingesetzt werden, dessen Brechzahl verschieden ist von den Brechzahlen der Einzelschichten des Pigmentes, so werden für eine optimale Farbeinstellung die Schichtdicken der Einzelschichten aus lichtdurchlässigem Material mit einer Brechzahl von n > 1,8 gegebenenfalls voneinander abweichen müssen. Diese Abweichung kann bis zu 50 %, bezogen auf die Schichtdicke der zuerst aufgetragenen Schicht mit einer Brechzahl > 1,8, betragen und lässt sich über fachgemäße Optimierungsarbeiten, die keines erfinderischen Zutuns bedürfen, leicht ermitteln. Gleichermassen kann eine Anpassung erfolgen, wenn Farbzwischentöne oder besonders steile Kanten der Reflexbanden des Pigmentes für bestimmte Anwendungsgebiete erwünscht sind.

Die besten Ergebnisse lassen sich jedoch mit Pigmenten gemäß der vorliegenden Erfindung erreichen, die einen völlig symmetrischen Schichtaufbau aufweisen. Dabei bestehen die einzelnen Schichten aus einem lichtdurchlässigen Material mit einer Brechzahl von ≤ 1,8 aus demselben Material und weisen dieselben Schichtdicken auf. Ebenso weisen die einzelnen Schichten aus einem lichtdurchlässigen Material mit einer Brechzahl von n > 1,8 dieselben Schichtdicken auf und bestehen aus demselben Material. Dabei ist es besonders bevorzugt, wenn das Substrat und die Schichten aus einem lichtdurchlässigen Material mit einer Brechzahl von ≤ 1,8 nicht nur dieselben optischen Schichtdicken aufweisen, sondern ebenfalls aus demselben Material, nämlich SiO₂, und die einzelnen Schichten aus einem lichtdurchlässigen Material mit einer Brechzahl von n > 1,8 aus TiO₂ bestehen.
Im letzteren Falle erhält man ein symmetrisches Pigment mit dem Schichtaufbau

TiO₂-SiO₂-TiO₂-SiO₂-TiO₂- SiO₂(-Substrat)-TiO₂-SiO₂-TiO₂-SiO₂-TiO₂,

welches ein Pigment mit elf optisch aktiven Schichten darstellt, obwohl das Substrat nur 5fach beschichtet wurde. Durch nachfolgende Beschichtung mit je einer weiteren Schicht TiO₂ und SiO₂ kann die Schichtfolge beliebig erweitert werden. Dabei haben alle Schichten aus denselben Materialien auch dieselben Schichtdicken. Die Anzahl der Schichten wird hierbei von den erzielbaren Farbeffekten und dem dafür nötigen Aufwand beschränkt.

In der Regel reicht der oben beschriebene Schichtaufbau aus, um Pigmente mit klaren und brillanten Interferenzfarben, starker Abhängigkeit der Interferenzfarben vom Betrachtungswinkel sowie einem gleichmäßig starken Buntton (Farbtiefe) bei allen winkelabhängig durchlaufenen Interferenzfarben zu erhalten.

Es hat sich überraschend gezeigt, dass bereits der Schichtaufbau

TiO₂-SiO₂-TiO₂- SiO₂(-Substrat)-TiO₂-SiO₂-TiO₂

zu außergewöhnlich guten Pigmenten mit den oben beschriebenen Eigenschaften führt, wobei zum Erhalt eines Pigmentes mit sieben optisch aktiven Schichten das Substrat nur mit drei Schichten beschichtet ist.

Von besonderem Interesse und sehr überraschend war die Tatsache, dass dieses Pigment einen Farbflop zeigt, den bereits das Pigment der Zusammensetzung

TiO₂- SiO₂(-Substrat)-TiO₂

aufweist, welches nur drei optisch aktive Schichten besitzt, deren tatsächliche einzelne Schichtdicken jeweils identisch sind mit den einzelnen Schichtdicken des Substrates und der ersten Beschichtung der Pigmente mit sieben oder elf optisch aktiven Schichten. Dabei kann die Spannweite des im L,a,b - Farbsystem nach Hunter sichtbaren Farbflops bei höherer Anzahl von Schichten geringfügig breiter sein, es werden jedoch dieselben Farbtöne abhängig vom Betrachtungswinkel beobachtet wie beim Dreischichtpigment gleicher Schichtdicken. Durch die zusätzliche Beschichtung mit weiteren Schichten gleicher Zusammensetzung und Schichtdicke lässt sich aber der Buntton der Pigmente auffällig verändern, das heißt verstärken, und zwar für jeden Farbton, der abhängig vom Betrachtungswinkel durchlaufen wird. Auch die Farbbrillanz ist bei den erfindungsgemäßen mehrschichtigen Interferenzpigmenten deutlich stärker ausgeprägt als bei den entsprechenden Dreischichtpigmenten.

Das oben Gesagte trifft selbstverständlich auch für die erfindungsgemäßen Pigmente zu, die nicht ausschließlich aus SiO₂ und TiO₂ zusammengesetzt sind, sondern in den einzelnen Schichten ein oder mehrere der oben als geeignet beschriebenen anderen Materialien enthalten und einen symmetrischen Aufbau besitzen, der bezüglich der Schichtdicken und Schichtdickentoleranzen in den oben beschriebenen Grenzen liegt.

Das erfindungsgemäße Verfahren zur Herstellung eines mehrschichtigen Interferenzpigmentes ist bevorzugt ein nasschemisches Verfahren, bei welchem die bekannten, zur Herstellung von Perlglanzpigmenten entwickelten nasschemischen Beschichtungstechnologien angewendet werden können, die zum Beispiel in den folgenden Publikationen beschrieben sind:
DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017.
Zur Beschichtung wird das plättchenförmige Substrat aus Siliziumdioxid in Wasser suspendiert und abwechselnd mehrfach mit einem Metalloxidhydrat eines Metalloxides oder mit BiOCI mit einer Brechzahl von jeweils n > 1,8 und einem Metalloxidhydrat eines Metalloxides oder einem Metallfluorid mit einer Brechzahl von n ≤ 1,8 durch Zugabe und Fällung der entsprechenden anorganischen Metallverbindungen beschichtet, wobei durch gleichzeitige Zugabe von Säure oder Base der für die Fällung des jeweiligen Metalloxidhydrates, BiOCI oder Metallfluorides notwendige pH-Wert eingestellt und konstant gehalten wird und anschließend das beschichtete Substrat aus der wässrigen Suspension abgetrennt, getrocknet und gegebenenfalls kalziniert wird, und wobei bei den einzelnen auf dem Substrat aufzubringenden Schichten aus einem Metalloxidhydrat eines Metalloxides oder aus Metallfluorid mit einer Brechzahl von n ≤ 1,8 eine optische Schichtdicke so eingestellt wird, dass sie nach dem Trocknen und gegebenenfalls Kalzinieren gleich der optischen Schichtdicke des plättchenförmigen Substrates ist.
Dabei kann die Kalzinierungstemperatur im Hinblick auf die jeweils vorhandene Beschichtung optimiert werden. Im Allgemeinen liegt die Kalzinierungstemperatur jedoch zwischen 250 und 1000 °C, insbesondere zwischen 350 und 900 °C. Die Pigmente können auch nach dem Aufbringen jeder einzelnen Schicht abgetrennt, getrocknet und gegebenenfalls geglüht werden, bevor sie zur Aufbringung der nächsten Schicht erneut dispergiert werden.

Wenn das Material mit einer Brechzahl n > 1,8 TiO₂ ist, wird zum Aufbringen dieser Schichten bevorzugt das im US 3 553 001 beschriebene Verfahren eingesetzt. Dabei wird zu einer auf etwa 50-100 °C, insbesondere 70 - 80 °C erhitzten Suspension der plättchenförmigen, gegebenenfalls bereits vorbeschichteten Siliziumdioxidpartikel langsam eine wässrige Lösung eines anorganischen Titansalzes zugegeben und der pH-Wert durch gleichzeitiges Zudosieren einer Base bei 0,5 bis 5, insbesondere etwa 1,5 bis 2,5, weitgehend konstant gehalten. Sobald die gewünschte Schichtdicke des TiO₂-Oxidhydrates erreicht ist, wird die Zugabe der Titansalzlösung und der Base gestoppt.
Dieses Verfahren wird auch als Titrationsverfahren bezeichnet und weist die Besonderheit auf, dass kein Überschuss an Titansalz vorliegt, sondern pro Zeiteinheit immer nur eine solche Menge bereitgestellt wird, wie für eine gleichmäßige Beschichtung mit dem hydratisierten TiO₂ erforderlich ist und auch von der Oberfläche des zu beschichtenden SiO₂-Substrates aufgenommen werden kann. In der Lösung sind daher keine hydratisierten Titandioxidteilchen vorhanden, die nicht auf der zu beschichtenden Oberfläche abgeschieden werden.

Wenn das lichtdurchlässige Material mit einer Brechzahl n ≤ 1,8 Siliziumdioxid ist, wird zum Aufbringen der entsprechenden Schicht oder Schichten das folgende Verfahren angewandt:
Eine Natronwasserglaslösung wird zu einer auf etwa 50 bis 100 °C, insbesondere 70 bis 80 °C, erhitzten Suspension des bereits ein- oder mehrfach beschichteten Substrates gegeben. Gleichzeitig wird durch Zugabe einer 10%igen Salzsäure der pH-Wert bei 4 bis 10, vorzugsweise bei 6,5 bis 8,5, konstant gehalten. Wenn die Zugabe der Wasserglaslösung beendet ist, wird noch etwa 30 Minuten nachgerührt.

Prinzipiell sind für die Herstellung der erfindungsgemäßen Pigmente auch CVD - oder PVD - Verfahren zur Beschichtung von Partikeln geeignet. Dabei ist es erforderlich, dass das Substrat während des Bedampfungsvorganges gleichmäßig in Bewegung gehalten wird, damit eine homogene Beschichtung aller Partikeloberflächen gewährleistet ist.

Werden auf der oberen Schicht aus Metalloxid oder BiOCI mit einer Brechzahl n > 1,8 noch zusätzlich weitere Schichten aus organischen oder anorganischen Farbpigmenten wie farbigen Metalloxiden, zum Beispiel Goethit, Magnetit, Hämatit, Chromoxid, Titansuboxiden und Chrom-Eisen-Mischoxiden, oder Farbpigmenten wie Berliner Blau, Turnbulls Blau, Bismutvanadat, Chromhydroxid, Cobaltaluminat, Ultramarin, Tenards Blau, Cadmiumsulfiden oder -seleniden, Chromatpigmenten oder Ruß, oder aber organischen Farbpigmenten wie Indigo, Thioindigo und deren Derivaten, Azopigmenten, Phthalocyaninen, Benzimidazolen, Anthrachinonen, Indanthrenfarbstoffen, Perinonen, Chinacridonen, Metallchalkogeniden, Metallchalkogenidhydraten oder Karminrot auf den erfindungsgemäßen Pigmenten aufgebracht, kann die Pulverfarbe der Pigmente wesentlich verändert werden, wodurch weitere interessante Farbeffekte erzielbar sind.

Das Aufbringen dieser Schichten erfolgt mittels bekannter Verfahren, wie sie zum Beispiel in EP 0 141 173, EP 0 332 071, DE 19 51 696, DE 19 51 697, DE 23 13 332 und DE 40 09 567 beschrieben sind.

Das fertige Pigment kann auch einer Nachbehandlung oder Nachbeschichtung unterzogen werden, um seine Licht-, Wetter-, oder chemische Stabilität zu erhöhen oder um die Handhabung des Pigmentes, insbesondere hinsichtlich der Einarbeitung in unterschiedliche Medien, zu erleichtern. Verfahren zur Nachbeschichtung oder Nachbehandlung sind zum Beispiel aus DE 22 15 191, DE 31 51 354, DE 32 35 017 DE 33 34 598, DE 40 30 727, EP 0 649 886, WO 97/29059, WO 99/57204 oder US 5,759, 255 bekannt.
Die hierbei aufgebrachten Stoffe umfassen lediglich einen Gewichtsanteil von 0,1 bis 5 Gew. %, vorzugsweise 0,5 bis 3 Gew. %, des gesamten Pigmentes.

Die erfindungsgemäßen Pigmente können zur Pigmentierung von Farben, Lacken, Druckfarben, Kunststoffen, kosmetischen Formulierungen, keramischen Materialien, Papier und Gläsern sowie in den verschiedenen Sicherheitsanwendungen in üblicher Weise eingesetzt werden. Weiterhin sind die erfindungsgemäßen Pigmente auch für die Lasermarkierung von Papier und Kunststoffen, für Anwendungen im Agrarbereich, sowie für die Herstellung von Pigmentpräparationen und Trockenpräparaten, wie z.B. Pellets, Granulaten, Chips, etc., die vorzugsweise in Druckfarben und Lacken verwendet werden, geeignet. Sie lassen sich ebenso in einer Vielzahl der bekannten, in Farbsystemen angewendeten Bindemittel einsetzen und sind sowohl in wässrigen Systemen als auch in Systemen auf Lösungsmittelbasis anwendbar.

Die Pigmente gemäß der vorliegenden Erfindung sind selbstverständlich in vorteilhafter Weise auch mit organischen Farbstoffen, organischen Pigmenten und jeder Art von anderen anorganischen Ein- oder Mehrschichtpigmenten, wie zum Beispiel den herkömmlichen Perlglanzpigmenten auf der Basis von Schichtsilikaten, Glas, SiO₂ oder Metallsubstraten, wie auch mit holographischen Pigmenten oder LCPs ( Liquid Crystal Polymers) mischbar und können gemeinsam mit diesen verwendet werden. Auch die Abmischung mit herkömmlichen Bindemitteln und Füllstoffen ist in jedem Verhältnis möglich.

Die erfindungsgemäßen mehrschichtigen Interferenzpigmente zeichnen sich durch eine starke Winkelabhängigkeit der Interferenzfarben aus, wobei alle in Abhängigkeit vom Beleuchtungs- oder Betrachtungswinkel beobachtbaren Farbtöne einen intensiven Buntton sowie eine hervorragende Farbbrillanz aufweisen. Sie sind mittels eines einfachen und kostengünstigen Verfahrens ohne besondere Sicherheitsmaßnahmen herstellbar, bei welchem bereits in der ersten Verfahrensstufe die Farben eingestellt werden können, die das fertige Pigment aufweisen soll.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung erläutern ohne sie zu beschränken.

### Beispiele

### Beispiel 1:

Herstellung eines Pigmentes der Zusammensetzung:
TiO₂ (145 nm)/ SiO₂ (200 nm)/ TiO₂ (145 nm) /SiO₂ (200 nm) / TiO₂ (145 nm/ SiO₂ (200 nm)/ TiO₂ (145 nm)
100 g SiO₂ Flakes mit einer mittleren Schichtdicke von 200 nm werden in 1,9 I voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (474 g einer 60 %igen TiCl₄ - Lösung , gelöst in 474 g voll entsalztem Wasser), wobei der pH Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH Wert der Suspension auf 7,5 eingestellt und erneut 15 min. nachgerührt.

Anschließend wird eine Natronwasserglaslösung (654 g NatronwasserglasLösung, enthaltend 27,0 % SiO₂, werden in 654 g voll entsalztem Wasser gelöst) zugetropft, wobei der pH Wert durch gleichzeitiges Zudosieren einer 18 %igen Salzsäure konstant bei 7,5 gehalten wird. Nach vollständiger Zugabe wird 30 min. nachgerührt.

Nun wird der pH Wert der Suspension mit 18 %iger Salzsäure auf 2,2 eingestellt, 30 min. nachgerührt und 949 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

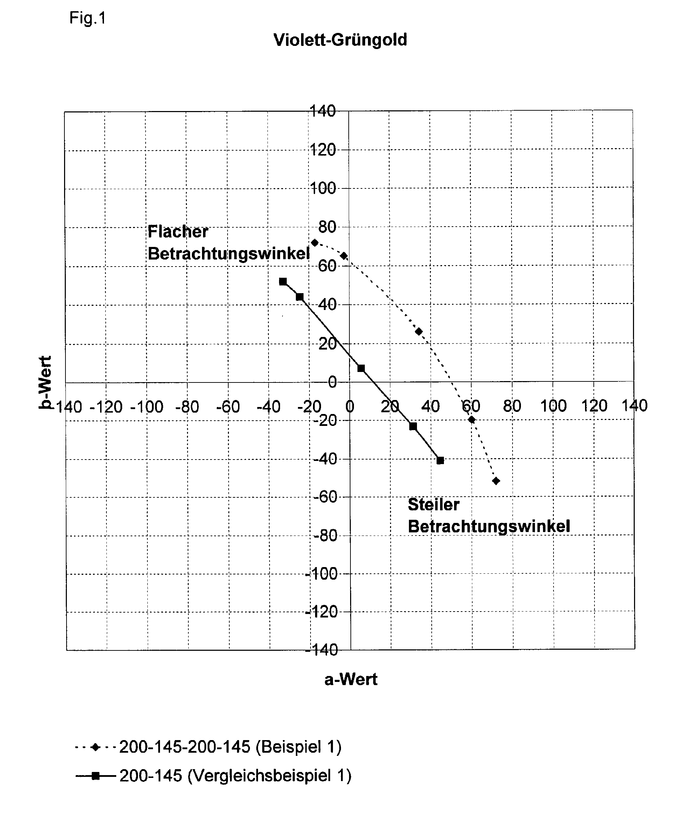

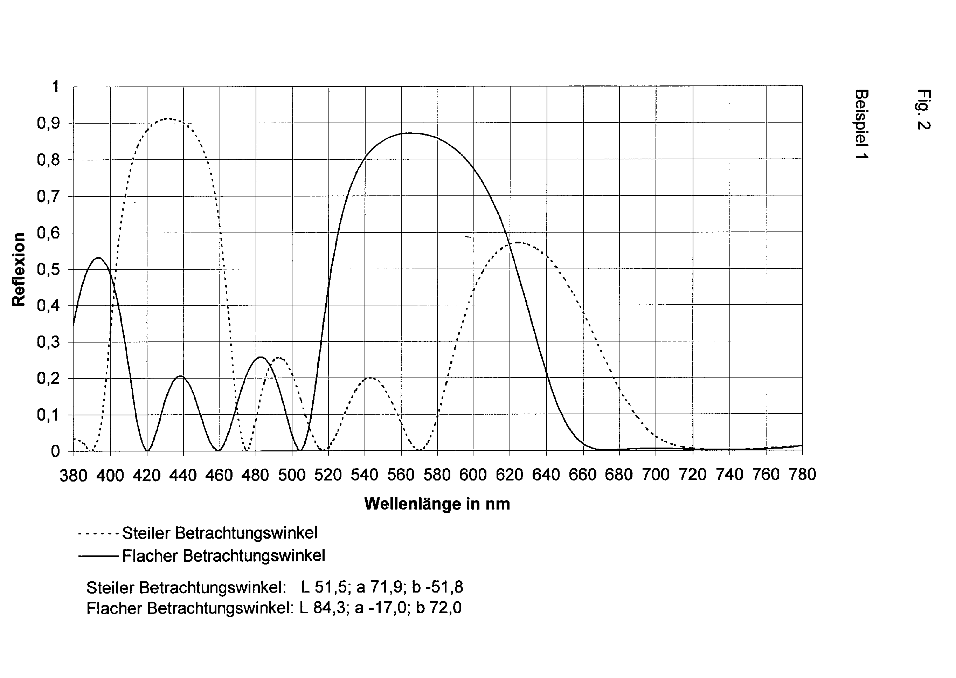

Das entsprechende L,a,b,-Farbdiagramm nach Hunter ist in Figur 1 mit gestrichelter Linie dargestellt. Es zeigt vom steilen zum flachen Betrachtungswinkel einen Farbflop von Violett nach Grüngold. Das Reflexionsspektrum (Reflexion in Abhängigkeit der Wellenlänge des sichtbaren Lichtes) ist in Figur 2 dargestellt.

### Vergleichsbeispiel 1:

Herstellung eines Pigmentes der Zusammensetzung
TiO₂ (145 nm)/ SiO₂ (200 nm)/ TiO₂ (145 nm)
100 g SiO₂ Flakes mit einer mittleren Schichtdicke von 200 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (474 g einer 60 %igen TiCl₄ - Lösung, gelöst in 474 g voll entsalztem Wasser), wobei der pH Wert durch gleichzeitiges Zutropfen einer 32 %igen Natronlauge konstant gehalten wird. Nach vollständiger Zugabe wird 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

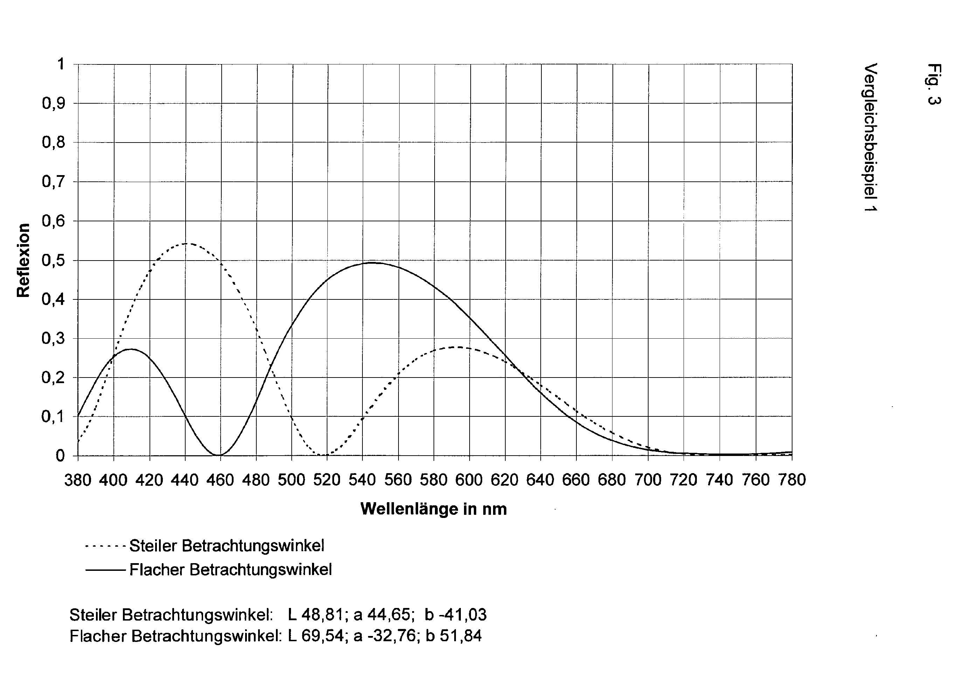

Das L,a,b,-Farbdiagramm nach Hunter für Vergleichsbeispiel 1 ist in Figur 1 mit durchgezogener Linie dargestellt. Es zeigt vom steilen zum flachen Betrachtungswinkel ebenfalls einen Farbflop von Violett nach Grüngold. Das Reflexionsspektrum ist in Figur 3 dargestellt.

Ein Vergleich der beiden Reflexionsspektren der Pigmente aus Beispiel 1 und Vergleichsbeispiel 1 zeigt, dass das Pigment gemäß Beispiel 1 der vorliegenden Erfindung ein deutlich verbessertes Reflexionsvermögen gegenüber einem dreischichtigen Pigment vergleichbarer Zusammensetzung aufweist.

### Beispiel 2:

Herstellung eines Pigmentes der Zusammensetzung:
TiO₂ (38 nm)/ SiO₂ (500 nm)/ TiO₂ (28 nm) /SiO₂ (500 nm) / TiO₂ (28 nm)/SiO₂ (500 nm)/ TiO₂ (38 nm)
100 g SiO₂ Flakes mit einer mittleren Schichtdicke von 500 nm werden in 1,9 l voll entsalztem Wasser unter Rühren suspendiert und auf 75 °C erhitzt.

Nun wird mit einer 18 %igen Salzsäure der pH -Wert der Suspension auf 2,2 eingestellt. Es folgt ein Zudosieren einer 30 %igen Titantetrachloridlösung (91,6 g einer 60 %igen TiCl₄ - Lösung, gelöst in 91,6 g voll entsalztem Wasser), wobei der pH Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH - Wert der Suspension auf 7,5 eingestellt und erneut 15 min. nachgerührt.

Anschließend wird eine Natronwasserglaslösung (965 g NatronwasserglasLösung, enthaltend 27,0 % SiO₂, werden in 965 g voll entsalztem Wasser gelöst) zugetropft, wobei der pH Wert durch gleichzeitiges Zudosieren einer 18 %igen Salzsäure konstant bei 7,5 gehalten wird. Nach vollständiger Zugabe wird 30 min. nachgerührt.

Nun wird der pH - Wert der Suspension mit 18 %iger Salzsäure auf 2,2 eingestellt, 30 min. nachgerührt und 249 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

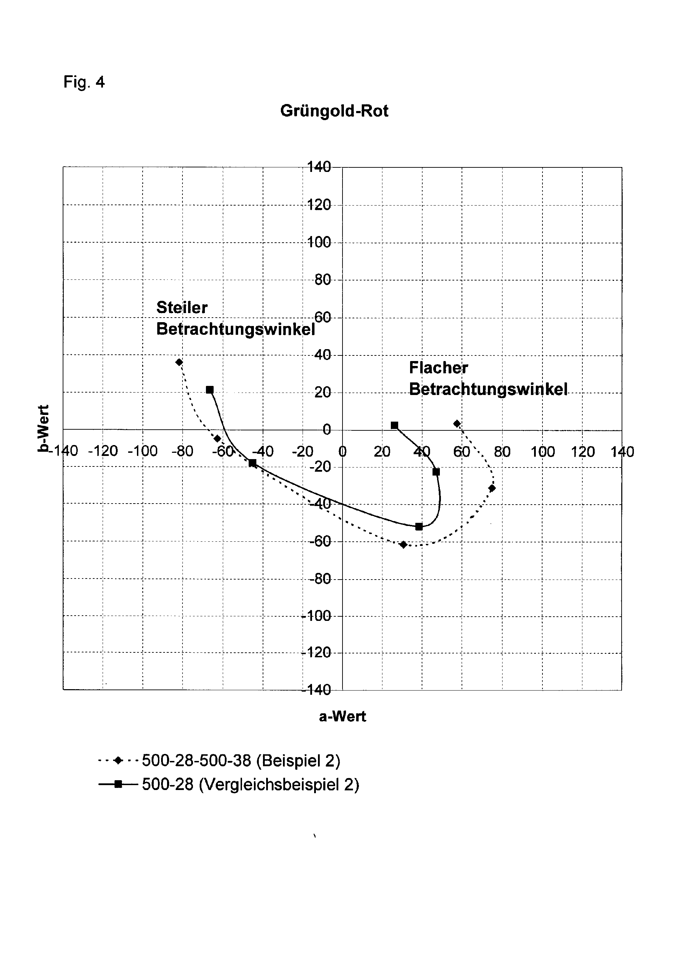

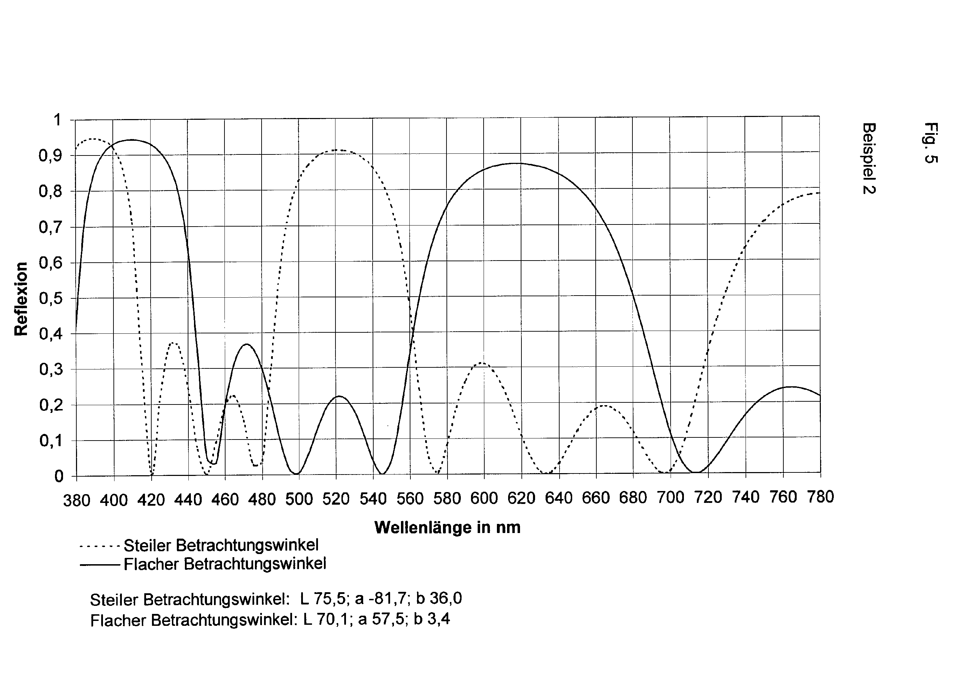

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen. Das erhaltene L,a,b-Diagramm ( siehe Figur 4, gestrichelte Linie) zeigt einen Farbflop von Grün/Gold im steilen Betrachtungswinkel nach Rot im flachen Betrachtungswinkel. Das Reflexionsspektrum ist in Figur 5 dargestellt.

### Vergleichsbeispiel 2:

Herstellung eines Pigmentes der Zusammensetzung
TiO₂ (28 nm)/ SiO₂ (500 nm)/ TiO₂ (28 nm)
100 g SiO₂ Flakes mit einer mittleren Schichtdicke von 500 nm werden in 1,9 l voll entsalztem Wasser unter Rühren suspendiert und auf 75 °C erhitzt.

Nun wird mit einer 18 %igen Salzsäure der pH -Wert der Suspension auf 2,2 eingestellt. Es folgt ein Zudosieren einer 30 %igen Titantetrachloridlösung (91,6 g einer 60 %igen TiCl₄ - Lösung, gelöst in 91,6 g voll entsalztem Wasser), wobei der pH Wert durch gleichzeitiges Zutropfen einer 32 %igen Natronlauge konstant gehalten wird. Nach vollständiger Zugabe wird 15 min. nachgerührt.

Das Produkt wird abfiltriert, gewaschen, getrocknet, bei 800°C geglüht, durch ein 100 um Sieb gesiebt, vom Pigment nach Einarbeitung in Nitrocellulose-Lack Lackkarten angefertigt und diese koloristisch vermessen.

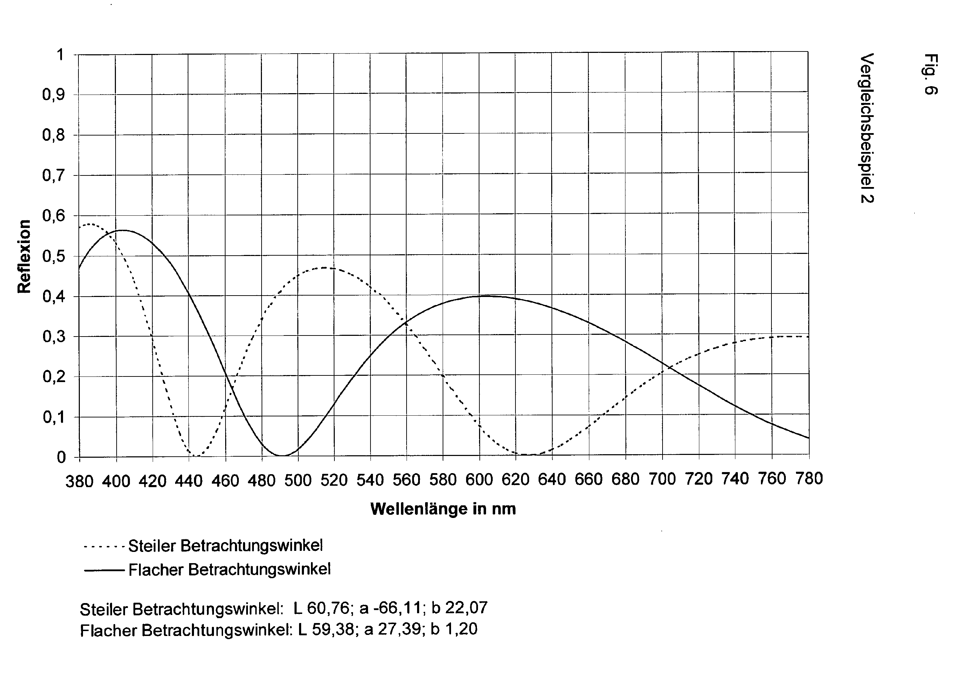

Das erhaltene L,a,b-Diagramm ( siehe Figur 4, durchgezogene Linie) zeigt wie bei Beispiel 2 einen Farbflop von Grün/Gold im steilen Betrachtungswinkel nach Rot im flachen Betrachtungswinkel. Das Reflexionsspektrum ist in Figur 6 dargestellt.

Ein Vergleich der Reflexionsspektren von Beispiel 2 und Vergleichsbeispiel 2 zeigt ein deutlich verbessertes Reflexionsvermögen des Pigmentes gemäß Beispiel 2.

### Beispiel 3:

Herstellung eines Pigmentes der Zusammensetzung:
TiO₂ (125 nm)/ SiO₂ (300 nm)/ TiO₂ (138 nm) /SiO₂ (300 nm) / TiO₂ (138 nm)/ SiO₂ (300 nm)/ TiO₂ (125 nm)
100 g SiO₂ Flakes mit einer mittleren Schichtdicke von 300 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (452 g einer 60 %igen TiCl₄ - Lösung, gelöst in 452 g voll entsalztem Wasser), wobei der pH - Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH - Wert der Suspension auf 7,5 eingestellt und erneut 15 min. nachgerührt.

Anschließend wird eine Natronwasserglaslösung (579 g NatronwasserglasLösung, enthaltend 27,3 % SiO₂, werden in 579 g voll entsalztem Wasser gelöst) zugetropft, wobei der pH - Wert durch gleichzeitiges Zudosieren einer 18 %igen Salzsäure konstant bei 7,5 gehalten wird. Nach vollständiger Zugabe wird 30 min. nachgerührt.

Nun wird der pH - Wert der Suspension mit 18 %iger Salzsäure auf 2,2 eingestellt, 30 min. nachgerührt und 818 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

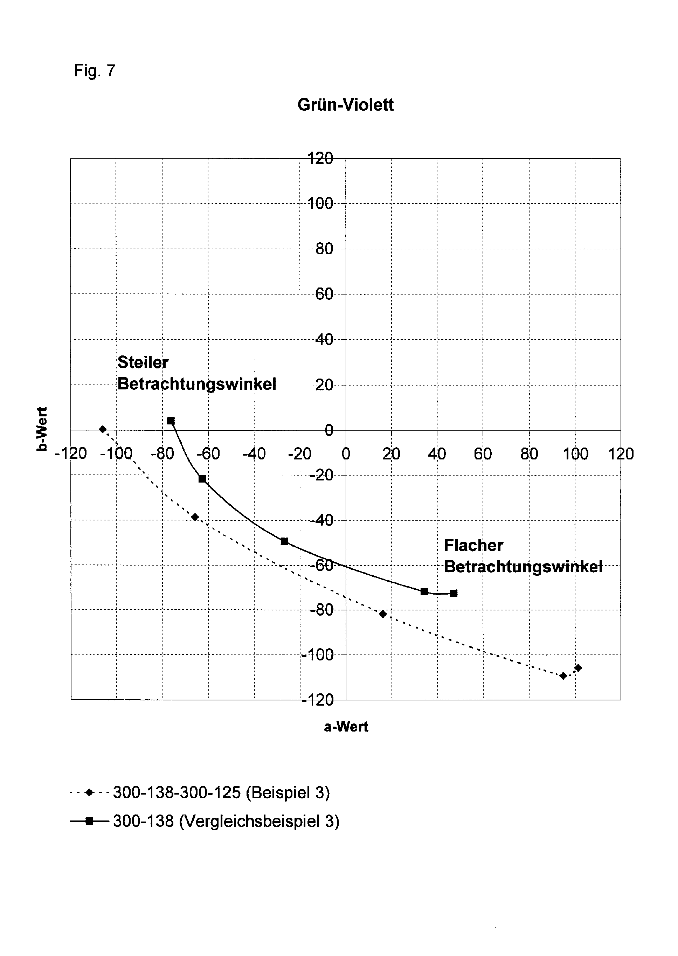

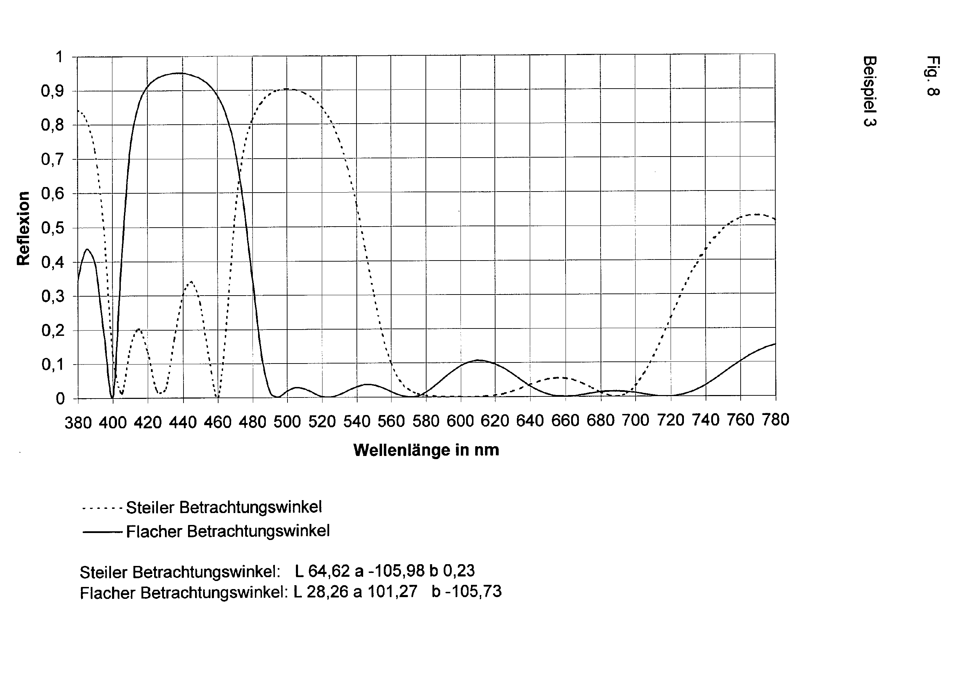

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen. Das erhaltene L,a,b-Diagramm ist in Figur 7 mit gestrichelter Linie dargestellt und zeigt einen Farbflop von Grün im steilen Betrachtungswinkel nach Violett im flachen Betrachtungswinkel. Das entsprechende Reflexionsspektrum wird durch Figur 8 wiedergegeben.

### Vergleichsbeispiel 3:

Herstellung eines Pigmentes der Zusammensetzung:
TiO₂ (138 nm) /SiO₂ (300 nm) / TiO₂ (138 nm)
100 g SiO₂ Flakes mit einer mittleren Schichtdicke von 300 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (452 g einer 60 %igen TiCl₄ - Lösung, gelöst in 452 g voll entsalztem Wasser), wobei der pH - Wert durch gleichzeitiges Zutropfen einer 32 %igen Natronlauge konstant gehalten wird. Nach vollständiger Zugabe wird 15 min. nachgerührt.

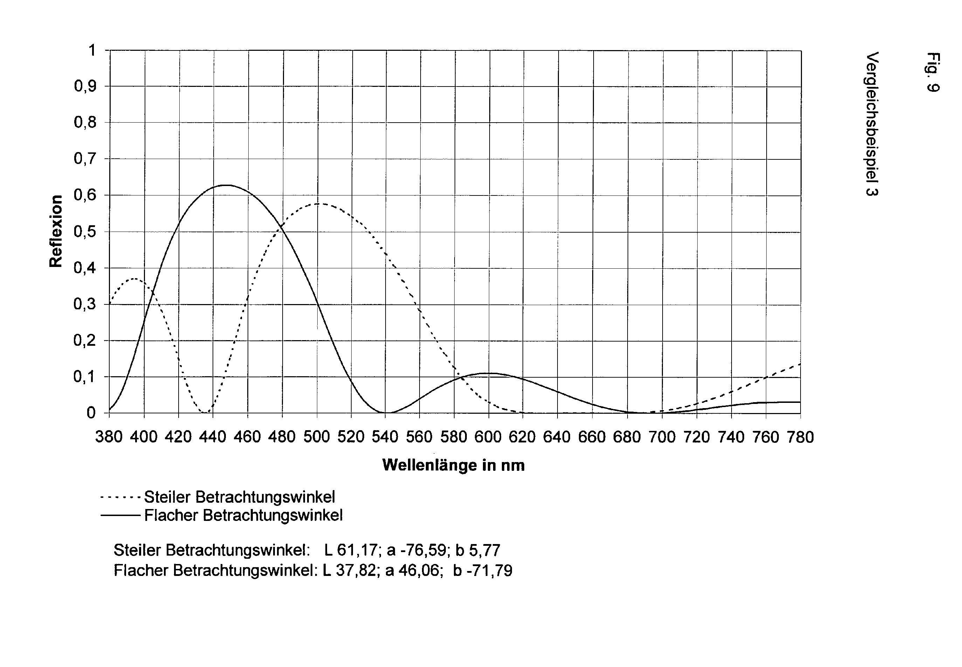

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen. Das erhaltene L,a,b-Diagramm ist in Figur 7 mit durchgezogener Linie dargestellt und zeigt ebenfalls einen Farbflop von Grün im steilen Betrachtungswinkel nach Violett im flachen Betrachtungswinkel. Das entsprechende Reflexionsspektrum wird durch Figur 9 wiedergegeben.

Ein Vergleich der Reflexionsspektren der Figuren 8 und 9 ergibt, dass das Pigment gemäß Beispiel 3 ein gegenüber Vergleichsbeispiel 3 deutlich verbessertes Reflexionsvermögen aufweist.

### Beispiel 4:

Herstellung eines Pigmentes der Zusammensetzung:
TiO₂ (73 nm)/ SiO₂ (300 nm)/ TiO₂ (70 nm) /SiO₂ (300 nm) / TiO₂ (70 nm)/SiO₂ (300 nm)/ TiO₂ (73 nm)
100 g SiO₂ Flakes mit einer mittleren Schichtdicke von 300 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (229 g einer 60 %igen TiCl₄ - Lösung, gelöst in 229 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH-Wert der Suspension auf 7,5 eingestellt und erneut 15 min. nachgerührt.

Anschließend wird eine Natronwasserglaslösung (579 g NatronwasserglasLösung, enthaltend 27,0 % SiO₂, werden in 579 g voll entsalztem Wasser gelöst) zugetropft, wobei der pH-Wert durch gleichzeitiges Zudosieren einer 18 %igen Salzsäure konstant bei 7,5 gehalten wird. Nach vollständiger Zugabe wird 30 min. nachgerührt.

Nun wird der pH-Wert der Suspension mit 18 %iger Salzsäure auf 2,2 eingestellt, 30 min. nachgerührt und 478 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

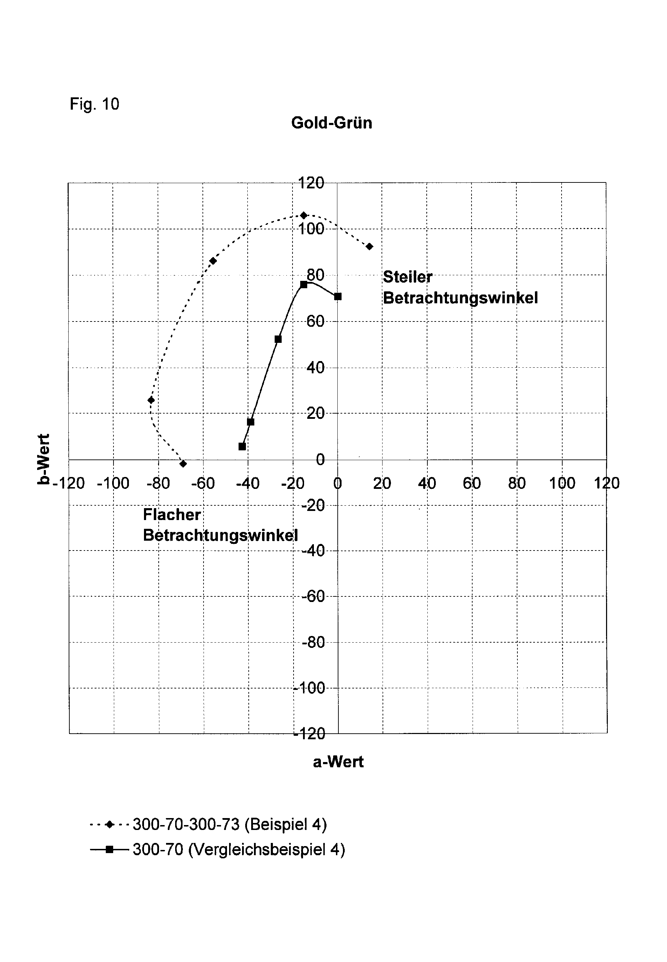

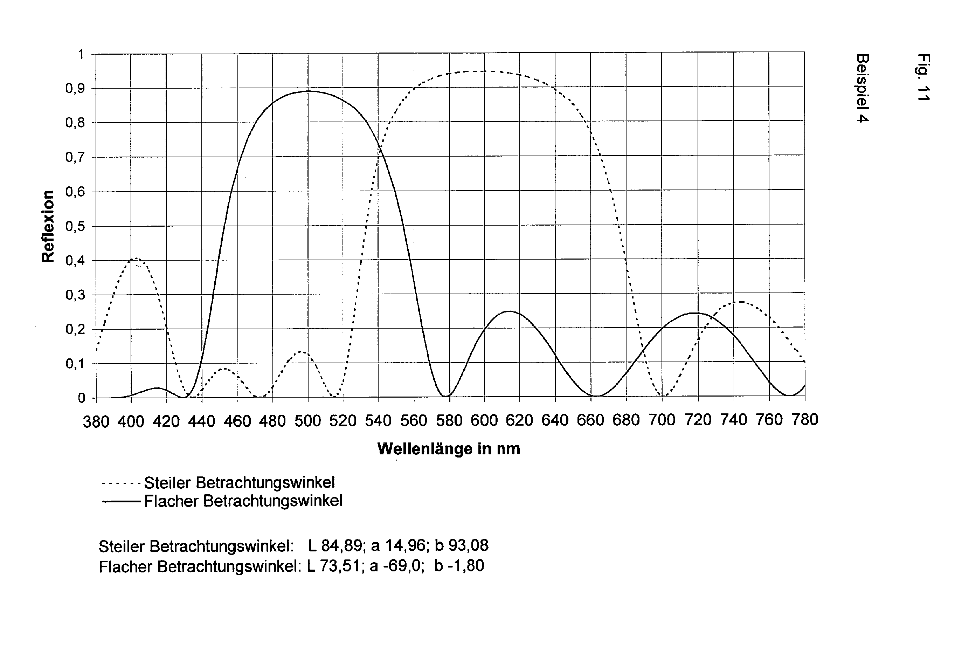

Das erhaltene L,a,b-Diagramm (siehe Figur 10, gestrichelte Linie) zeigt einen Farbflop von Gold im steilen Betrachtungswinkel nach Grün im flachen Betrachtungswinkel. Das Reflexionsspektrum ist in Figur 11 dargestellt.

### Verqleichsbeispiel 4:

Herstellung eines Pigmentes der Zusammensetzung:
TiO₂ (70 nm) /SiO₂ (300 nm) / TiO₂ (70 nm)
100 g SiO₂ Flakes mit einer mittleren Schichtdicke von 300 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (229 g einer 60 %igen TiCl₄ - Lösung, gelöst in 229 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

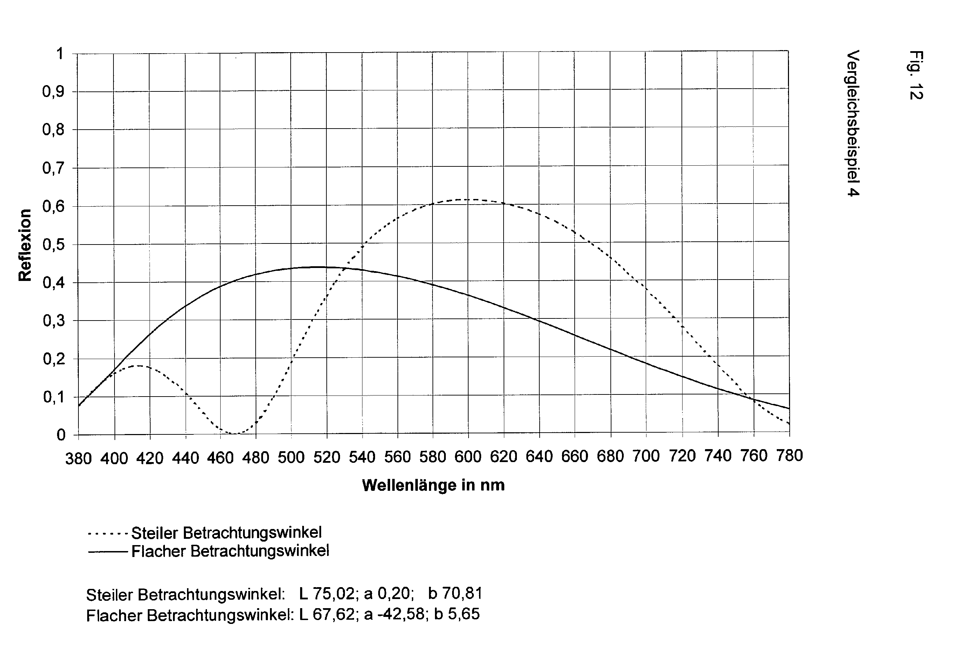

Das erhaltene L,a,b-Diagramm (siehe Figur 10, durchgezogene Linie) zeigt wie Beispiel 4 einen Farbflop von Gold im steilen Betrachtungswinkel nach Grün im flachen Betrachtungswinkel. Das Reflexionsspektrum ist in Figur 12 dargestellt.

Ein Vergleich der Reflexionsspektren des Pigmentes gemäß Beispiel 4 und des Pigmentes gemäß Vergleichsbeispiel 4 ergibt ein deutlich besseres Reflexionsvermögen für das erfindungsgemäße Beispiel.

### Beispiel 5:

Herstellung eines Pigmentes der Zusammensetzung:
TiO₂ (73 nm)/ SiO₂ (375 nm)/ TiO₂ (55 nm) /SiO₂ (375 nm) / TiO₂ (55 nm)/SiO₂ (375 nm)/ TiO₂ (73 nm)
100 g SiO₂ Flakes mit einer mittleren Schichtdicke von 375 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (180 g einer 60 %igen TiCl₄ - Lösung, gelöst in 180 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH-Wert der Suspension auf 7,5 eingestellt und erneut 15 min. nachgerührt.

Anschließend wird eine Natronwasserglaslösung (724 g NatronwasserglasLösung, enthaltend 27,0 % SiO₂, werden in 724 g voll entsalztem Wasser gelöst) zugetropft, wobei der pH-Wert durch gleichzeitiges Zudosieren einer 18 %igen Salzsäure konstant bei 7,5 gehalten wird. Nach vollständiger Zugabe wird 30 min. nachgerührt.

Nun wird der pH-Wert der Suspension mit 18 %iger Salzsäure auf 2,2 eingestellt, 30 min. nachgerührt und 478 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

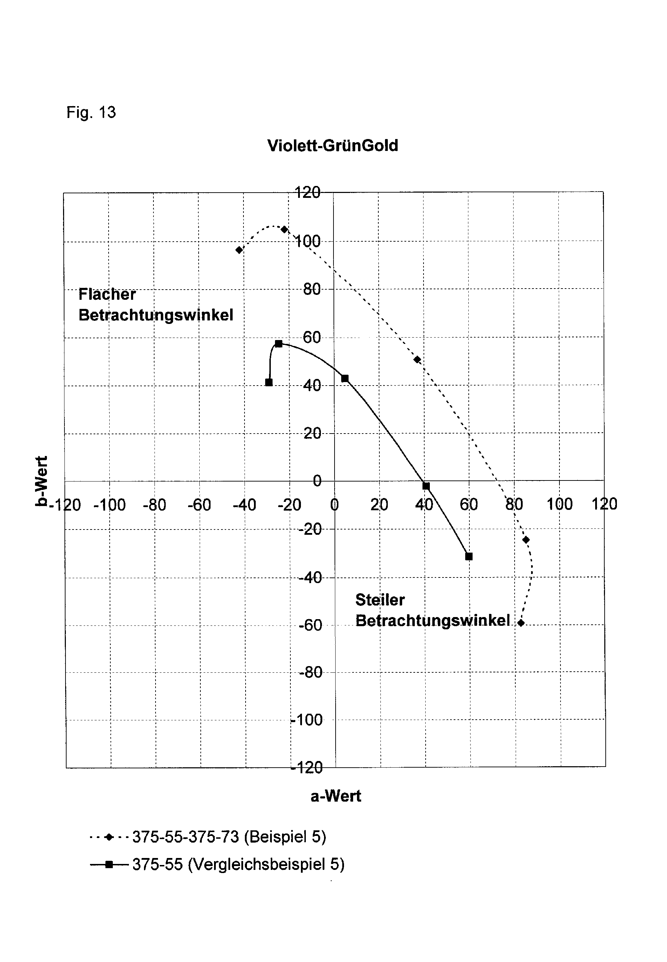

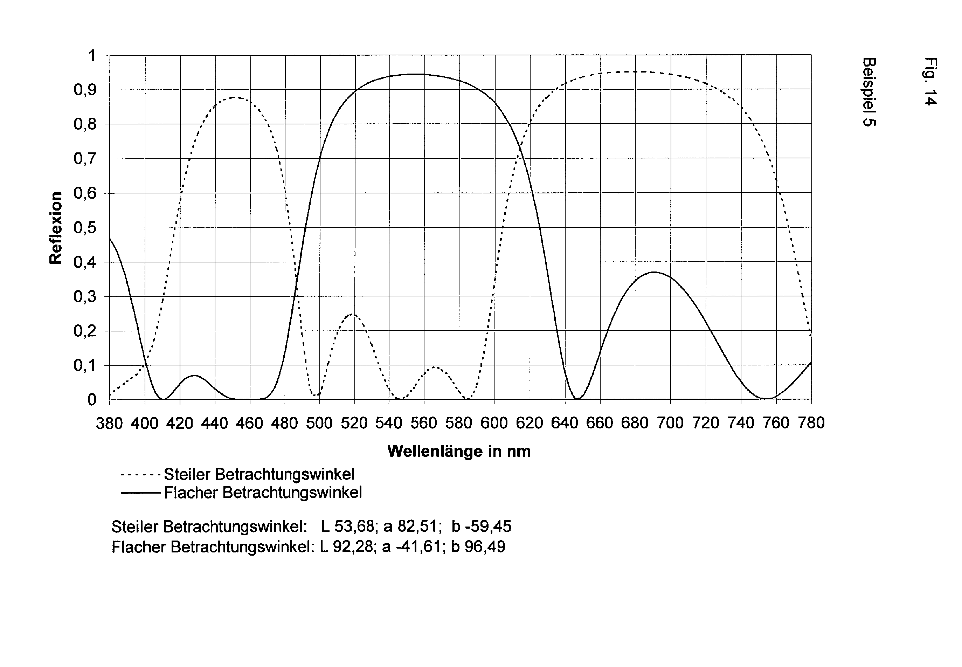

Das erhaltene L,a,b-Diagramm ist in Figur 13 mit gestrichelter Linie dargestellt und zeigt einen Farbflop von Violett im steilen Betrachtungswinkel nach Grüngold im flachen Betrachtungswinkel.
Das entsprechende Reflexionsspektrum zeigt Figur 14.

### Vergleichsbeispiel 5:

Herstellung eines Pigmentes der Zusammensetzung:
TiO₂ (55 nm) /SiO₂ (375 nm) / TiO₂ (55 nm)
100 g SiO₂ Flakes mit einer mittleren Schichtdicke von 375 nm werden in 1,9 I voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (180 g einer 60 %igen TiCl₄ - Lösung, gelöst in 180 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

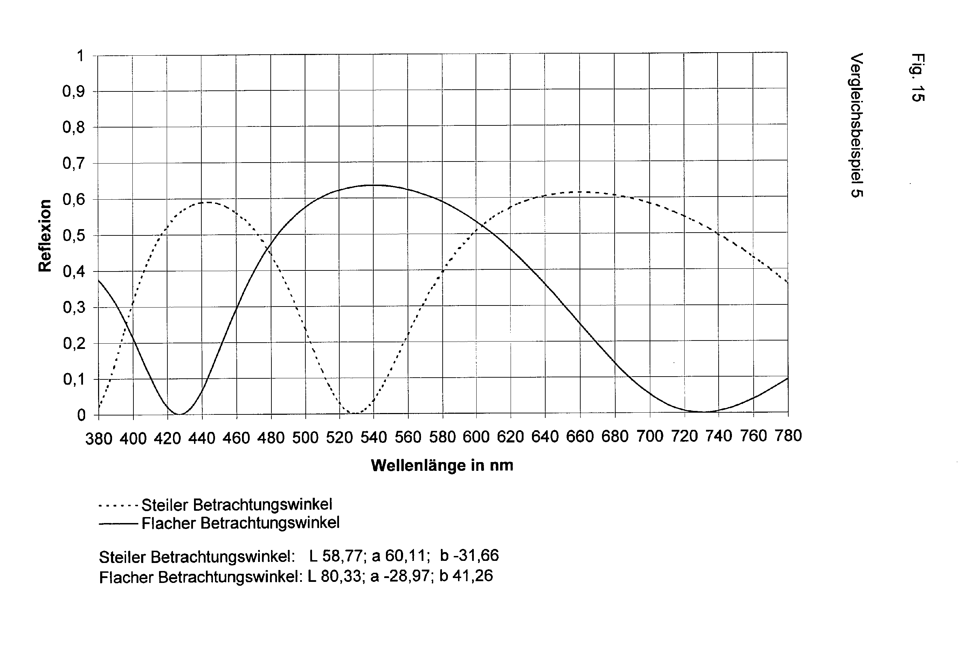

Das erhaltene L,a,b-Diagramm ist in Figur 13 mit durchgezogener Linie dargestellt und zeigt ebenfalls einen Farbflop von Violett im steilen Betrachtungswinkel nach Grüngold im flachen Betrachtungswinkel.
Das entsprechende Reflexionsspektrum zeigt Figur 15.

Ein Vergleich der Reflexionsspektren der Figuren 14 und 15 ergibt, dass das Pigment gemäß Beispiel 5 ein gegenüber Vergleichsbeispiel 5 deutlich verbessertes Reflexionsvermögen aufweist.

### Beispiel 6:

Herstellung eines Pigmentes der Zusammensetzung:
TiO₂ (48 nm)/ SiO₂ (280 nm)/ TiO₂ (48 nm) /SiO₂ (280 nm) / TiO₂ (48 nm)/SiO₂ (280 nm)/ TiO₂ (48 nm)
100 g SiO₂ Flakes mit einer mittleren Schichtdicke von 280 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (157 g einer 60 %igen TiCl₄ - Lösung, gelöst in 157 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH-Wert der Suspension auf 7,5 eingestellt und erneut 15 min. nachgerührt.

Anschließend wird eine Natronwasserglaslösung (540 g NatronwasserglasLösung, enthaltend 27,0 % SiO₂, werden in 540 g voll entsalztem Wasser gelöst) zugetropft, wobei der pH-Wert durch gleichzeitiges Zudosieren einer 18 %igen Salzsäure konstant bei 7,5 gehalten wird. Nach vollständiger Zugabe wird 30 min. nachgerührt.

Nun wird der pH-Wert der Suspension mit 18 %iger Salzsäure auf 2,2 eingestellt, 30 min. nachgerührt und 314 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

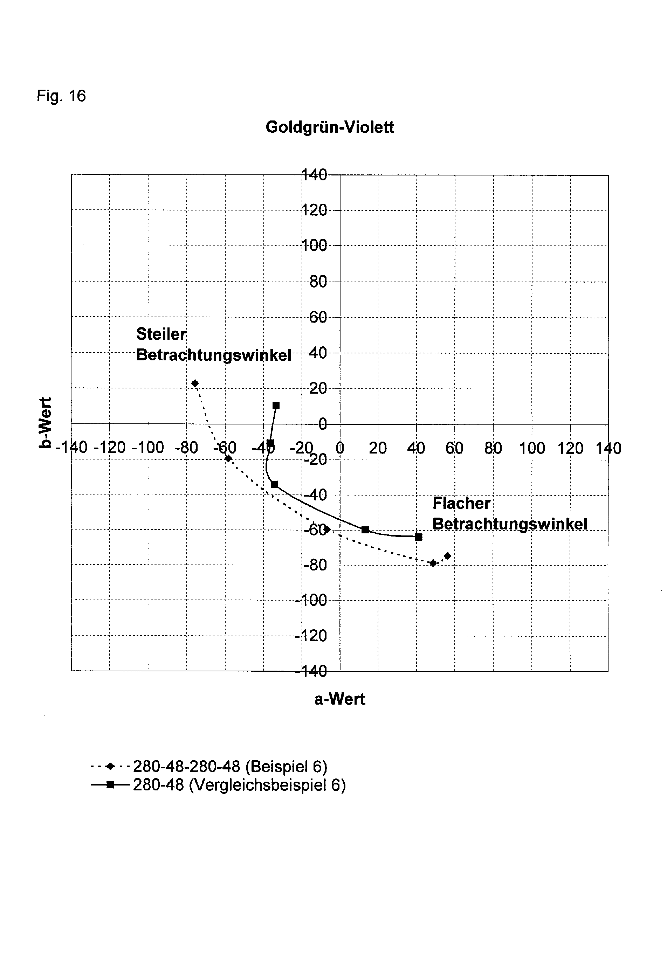

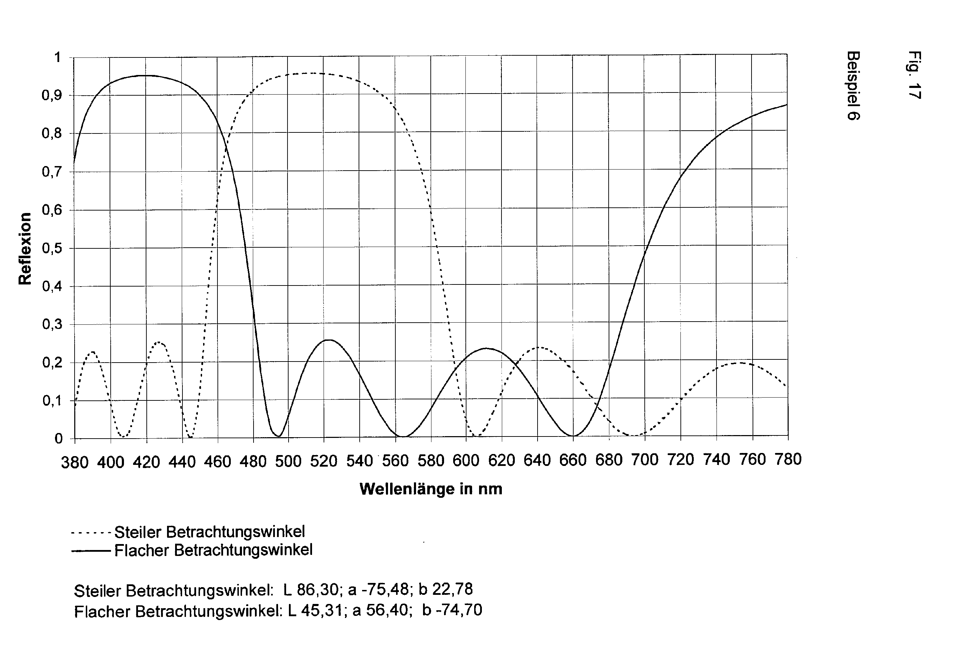

Das erhaltene L,a,b-Diagramm ist in Figur 16 mit gestrichelter Linie dargestellt und zeigt einen Farbflop von Grün-Gold im steilen Betrachtungswinkel über Blau-Grün nach Violett im flachen Betrachtungswinkel. Der Buntton der durchlaufenen Farbtöne ist dabei annähernd gleich gut ausgeprägt. Das Reflexionsspektrum ist in Figur 17 dargestellt.

### Vergleichsbeispiel 6:

Herstellung eines Pigmentes der Zusammensetzung:
TiO₂ (48 nm)/ SiO₂ (280 nm)/ TiO₂ (48 nm)
100 g SiO₂ Flakes mit einer mittleren Schichtdicke von 280 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (157 g einer 60 %igen TiCl₄ - Lösung, gelöst in 157 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

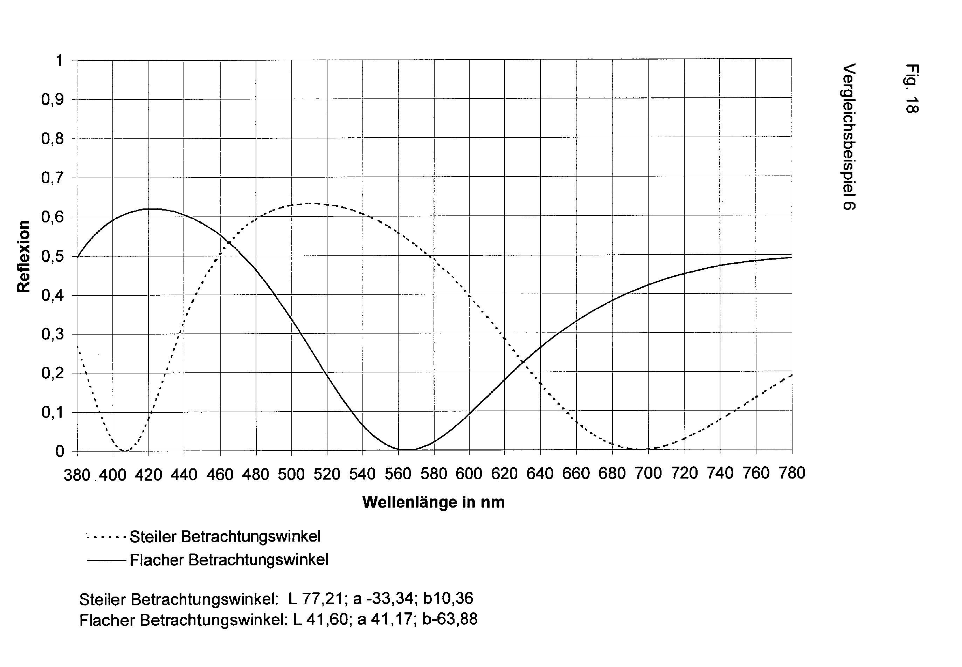

Das erhaltene L,a,b-Diagramm ist in Figur 16 mit durchgezogener Linie dargestellt und zeigt ebenfalls einen Farbflop von Grün-Gold im steilen Betrachtungswinkel über Blau-Grün nach Violett im flachen Betrachtungswinkel. Der Buntton der durchlaufenen Farbtöne ist dabei unterschiedlich stark ausgeprägt und insgesamt bei jedem Farbton schwächer als in Beispiel 6. Das entsprechende Reflexionsspektrum ist in Figur 18 dargestellt.

Ein Vergleich der Reflexionsspektren des Beispiels 6 und des Vergleichsbeispiels 6 zeigt ein deutlich besseres Reflexionsvermögen des Pigmentes gemäß Beispiel 6.

### Vergleichsbeispiel 7:

Herstellung eines unsymmetrischen Pigmentes gemäß Beispiel 7 aus DE 196 18 569, entspricht etwa dem Pigment gemäß Beispiel 6, jedoch mit unsymmetrischem Aufbau:
TiO₂ (48 nm)/ SiO₂ (173 nm)/ TiO₂ (33 nm) /SiO₂ (280 nm) / TiO₂ (33 nm)/SiO₂ (173 nm)/ TiO₂ (48 nm)
100 g SiO₂ Flakes mit einer mittleren Schichtdicke von 280 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (108 g einer 60 %igen TiCl₄ - Lösung, gelöst in 108 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Dann wird mit der 32%igen Natronlauge der pH-Wert der Suspension auf 7,5 eingestellt und erneut 15 min. nachgerührt.

Anschließend wird eine Natronwasserglaslösung (334 g NatronwasserglasLösung, enthaltend 27,0 % SiO₂, werden in 334 g voll entsalztem Wasser gelöst) zugetropft, wobei der pH-Wert durch gleichzeitiges Zudosieren einer 18 %igen Salzsäure konstant bei 7,5 gehalten wird. Nach vollständiger Zugabe wird 30 min. nachgerührt.

Nun wird der pH-Wert der Suspension mit 18 %iger Salzsäure auf 2,2 eingestellt, 30 min. nachgerührt und 314 g einer 30 %igen Titantetrachloridlösung (Herstellung s.o.) zugetropft. PH 2,2 wird hierbei durch Zutropfen einer 32 %igen Natronlauge konstant gehalten. Es wird nun erneut 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

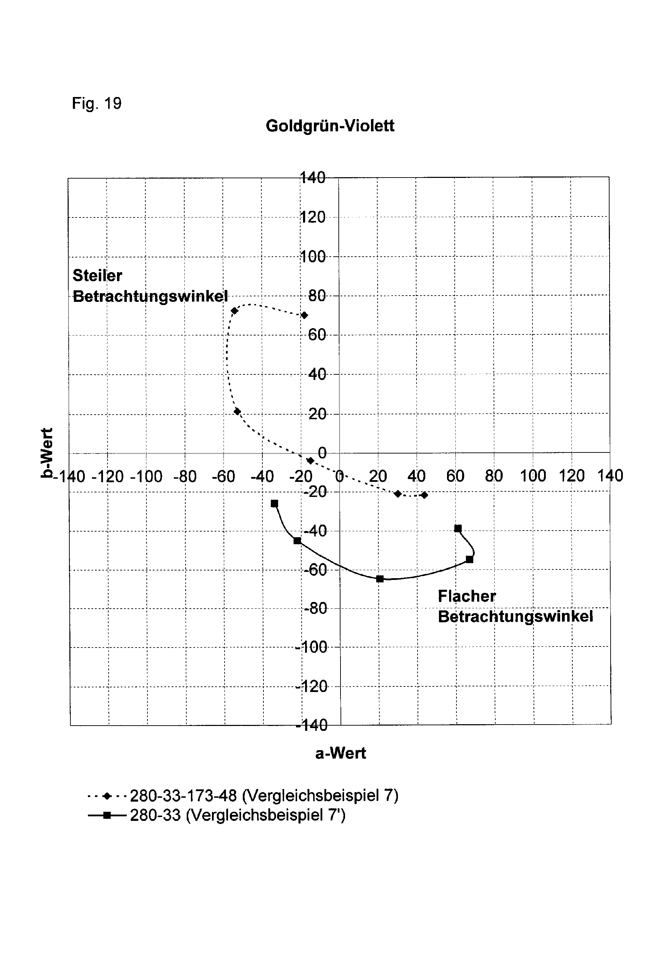

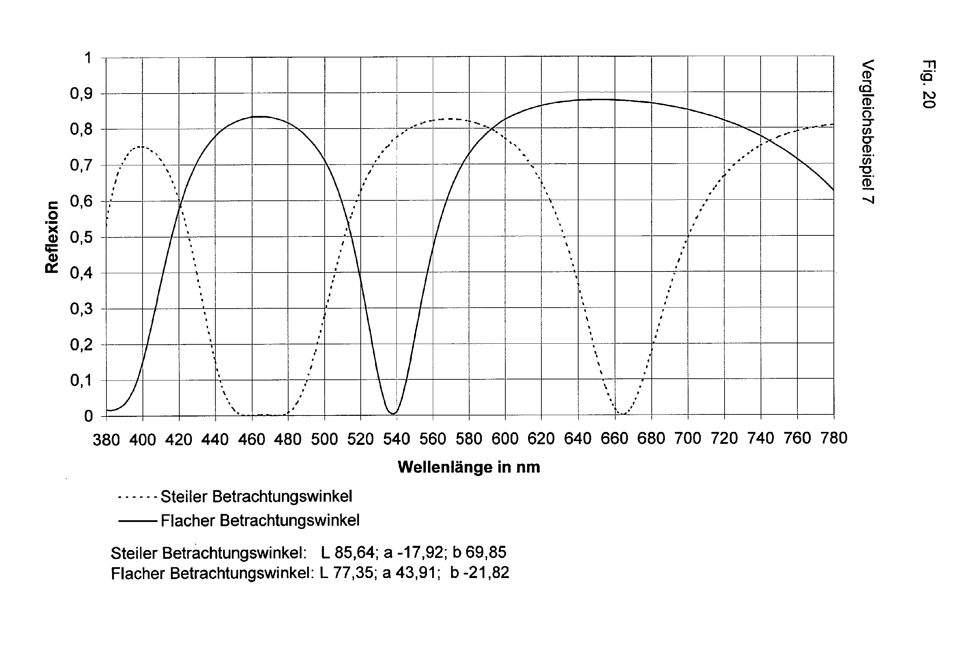

Das erhaltene L,a,b-Diagramm (siehe Figur 19, gestrichelte Linie) zeigt einen Farbflop von Gold-Grün im steilen Betrachtungswinkel über Blau-Grün nach Violett im flachen Betrachtungswinkel. Der Buntton ist jedoch bei den verschiedenen Farben sehr unterschiedlich ausgeprägt und lediglich im Bereich Gold-Grün ausreichend intensiv. Das Reflexionsspektrum ist in Figur 20 dargestellt.

### Verqleichsbeispiel 7':

Herstellung eines Pigmentes der Zusammensetzung:
TiO₂ (33 nm) /SiO₂ (280 nm) / TiO₂ (33 nm)
100 g SiO₂ Flakes mit einer mittleren Schichtdicke von 280 nm werden in 1,9 l voll entsalztem Wasser unter Rühren suspendiert und auf 75 °C erhitzt.

Nun wird mit einer 18 %igen Salzsäure der pH-Wert der Suspension auf 2,2 eingestellt. Es folgt ein Zudosieren einer 30 %igen Titantetrachloridlösung (108 g einer 60 %igen TiCl₄ - Lösung, gelöst in 108 g voll entsalztem Wasser), wobei der pH-Wert durch gleichzeitiges Zutropfen einer 32 %igen Natronlauge konstant gehalten wird. Nach vollständiger Zugabe wird 15 min. nachgerührt.

Das Produkt wird abfiltriert, gewaschen, getrocknet, bei 800°C geglüht, durch ein 100 µm Sieb gesiebt, vom Pigment nach Einarbeitung in Nitrocellulose-Lack Lackkarten angefertigt und diese koloristisch vermessen.

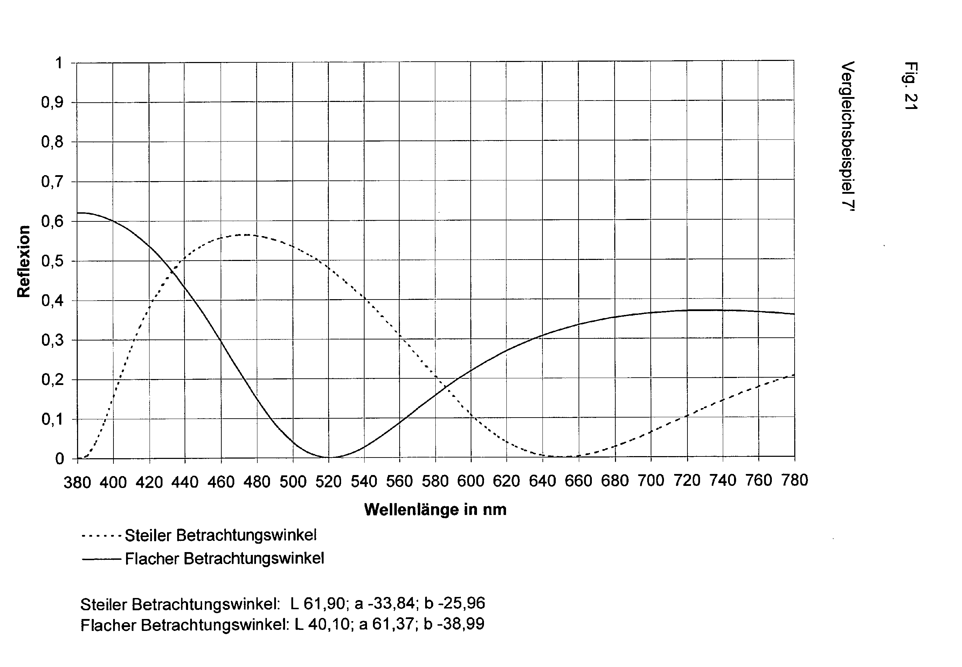

Das erhaltene L,a,b-Diagramm (siehe Figur 19, durchgezogene Linie) zeigt einen Farbflop von Blau-Grün im steilen Betrachtungswinkel nach Violett im flachen Betrachtungswinkel. Das Reflexionsspektrum ist in Figur 21 dargestellt.

Der Vergleich der Reflexionsspektren der Figuren 20 und 21 zeigt ein verbessertes Reflexionsvermögen des Pigmentes gemäß Vergleichsbeispiel 7.

Gleichzeitig wird durch den unsymmetrischen Aufbau allerdings der Farbflopverlauf gegenüber dem 3-schichtigen System gemäß Vergleichsbeispiel 7' vollständig verschoben (siehe Figur 19). Während die intensivste Farbe des Pigmentes gemäß Vergleichsbeispiel 7 im Gold-Grün-Bereich liegt, ist bei Vergleichsbeispiel 7' der Gold-Grün-Ton nicht vorhanden und der Violettton am besten ausgeprägt.

Das unsymmetrische Pigment gemäß Vergleichsbeispiel 7 zeigt damit gegenüber dem vergleichbaren symmetrischen Pigment gemäß Beispiel 6 einen völlig verschiedenen Verlauf des Farbflops, wobei das Pigment gemäß Beispiel 6 im Gegensatz zu Vergleichsbeispiel 7 zusätzlich gleichmäßig gute Farbtöne in allen durchlaufenen Farbbereichen aufweist.

## Patentansprüche

1. Mehrschichtiges Interferenzpigment, umfassend ein Substrat aus plättchenförmigem Siliziumdioxid, welches abwechselnd mit zwei oder mehreren Schichten aus lichtdurchlässigen Materialien mit Brechzahlen von n > 1,8 und einer oder mehreren Schichten aus lichtdurchlässigen Materialien mit Brechzahlen von n ≤ 1,8 beschichtet ist, wobei die optische Dicke des plättchenförmigen Substrates und die optische Dicke der einzelnen Schichten aus lichtdurchlässigen Materialien mit Brechzahlen von n ≤ 1,8 gleich ist.

2. Mehrschichtiges Interferenzpigment gemäß Anspruch 1, welches mit zwei Schichten aus lichtdurchlässigen Materialien mit Brechzahlen von n > 1,8 und einer Schicht aus lichtdurchlässigen Materialien mit Brechzahlen von n ≤ 1,8 beschichtet ist.

3. Mehrschichtiges Interferenzpigment gemäß Anspruch 1 oder 2, wobei das lichtdurchlässige Material mit Brechzahlen von n > 1,8 TiO₂, ZrO₂, ZnO, SnO₂, ein diese enthaltendes Gemisch, oder BiOCI ist.

4. Mehrschichtiges Interferenzpigment gemäß Anspruch 3, wobei das lichtdurchlässige Material mit einer Brechzahl von n > 1,8 TiO₂ ist.

5. Mehrschichtiges Interferenzpigment gemäß einem oder mehreren der Ansprüchen 1 bis 4, wobei das lichtdurchlässige Material mit Brechzahlen von n ≤ 1,8 SiO₂, SiO(OH)₂, Al₂O₃, AIOOH, B₂O₃, MgF₂ oder ein diese enthaltendes Gemisch ist.

6. Mehrschichtiges Interferenzpigment gemäß Anspruch 5, wobei das lichtdurchlässige Material mit einer Brechzahl von n ≤ 1,8 SiO₂ ist.

7. Mehrschichtiges Interferenzpigment gemäß einem oder mehreren der Ansprüche 1 bis 6, wobei das plättchenförmige Substrat und die einzelnen Schichten aus lichtdurchlässigen Materialien mit Brechzahlen von n ≤ 1,8 Schichtdicken zwischen 100 und 600 nm aufweisen.

8. Mehrschichtiges Interferenzpigment gemäß einem oder mehreren der Ansprüche 1 bis 7, wobei die einzelnen Schichten aus lichtdurchlässigen Materialien mit Brechzahlen von n > 1,8 Schichtdicken zwischen 20 und 300 nm aufweisen.

9. Mehrschichtiges Interferenzpigment gemäß Anspruch 8, wobei die Schichtdicken der einzelnen Schichten aus lichtdurchlässigen Materialien mit Brechzahlen von n > 1,8 gleich sind.

10. Mehrschichtiges Interferenzpigment gemäß einem oder mehreren der Ansprüche 1 bis 9, wobei die einzelnen Schichten aus lichtdurchlässigen Materialien mit Brechzahlen von n ≤ 1,8 größere Schichtdicken aufweisen als die einzelnen Schichten aus lichtdurchlässigen Materialien mit Brechzahlen von n > 1,8.

11. Verfahren zur Herstellung eines mehrschichtigen Pigmentes gemäß.einem oder mehreren der Ansprüche 1 bis 10, wobei das plättchenförmige Siliziumdioxidsubstrat in Wasser suspendiert und abwechselnd mehrfach mit einem Metalloxidhydrat oder BiOCI mit einer Brechzahl von n > 1,8 und einem Metalloxidhydrat oder Metallfluorid mit einer Brechzahl von n ≤ 1,8 durch Zugabe und Fällung der entsprechenden, anorganischen Metallverbindungen beschichtet wird, wobei durch gleichzeitige Zugabe von Säure oder Base der für die Fällung des jeweiligen Metalloxidhydrates, BiOCl oder Metallfluorides notwendige pH-Wert eingestellt und konstant gehalten wird und anschließend das beschichtete Substrat aus der wässrigen Suspension abgetrennt, getrocknet und gegebenenfalls kalziniert wird, und wobei bei den einzelnen auf dem Substrat aufzubringenden Schichten aus einem Metalloxidhydrat oder Metallfluorid mit einer Brechzahl von n ≤ 1,8 eine optische Schichtdicke derart eingestellt wird, dass sie nach dem Trocknen und gegebenenfalls Kalzinieren gleich der optischen Schichtdicke des plättchenförmigen Substrates ist.

12. Verwendung des mehrschichtigen Pigmentes gemäß einem oder mehreren der Ansprüche 1 bis 10 in Farben, Lacken, Druckfarben, Kunststoffen, kosmetischen Formulierungen, keramischen Materialien, Papier, Folien, Verpackungsmaterialien, Gläsern, Pigmentpräparationen, Trockenpräparaten, in Sicherheitsanwendungen sowie zur Lasermarkierung.

13. Farben, Lacke, Druckfarben, Kunststoffe, kosmetische Formulierungen, keramische Materialien, Papier, Folien, Verpackungsmaterialien, Gläser, Pigmentpräparationen, Trockenpräparate und Materialien für Sicherheitsanwendungen, enthaltend ein Pigment gemäß einem oder mehreren der Ansprüche 1 bis 10.
